(19) Europäisches Patentamt
European Patent Office
Office européen des brevets

(11) Veröffentlichungsnummer: **0 177 870 B1**

(12) # EUROPÄISCHE PATENTSCHRIFT

(45) Veröffentlichungstag der Patentschrift: **10.04.91**

(51) Int. Cl.⁵: **A61K 31/54**, A61K 47/18

(21) Anmeldenummer: **85112378.6**

(22) Anmeldetag: **30.09.85**

Die Akte enthält technische Angaben, die nach dem Eingang der Anmeldung eingereicht wurden und die nicht in dieser Patentschrift enthalten sind.

(54) **Stabilisierte Injektionslösungen von Piroxicam.**

(30) Priorität: **10.10.84 DE 3437232**

(43) Veröffentlichungstag der Anmeldung:
**16.04.86 Patentblatt 86/16**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**EP-A- 0 093 999**
**BE-A- 629 650**
**DE-A- 2 602 363**
**FR-A- 2 535 205**

(73) Patentinhaber: **Heinrich Mack Nachf.**
**Postfach 140**
**W-7918 Illertissen(DE)**

(72) Erfinder: **Fries, Walter**
**Einsteinring 25**
**W-7918 Illertissen(DE)**

(74) Vertreter: **Lederer, Franz, Dr. et al**
**Lederer, Keller & Riederer Patentanwälte**
**Lucile-Grahn-Strasse 22**
**W-8000 München 80(DE)**

EP 0 177 870 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Piroxicam, das ist 4-Hydroxy-2-methyl-N-2-pyridyl-l-2H-1,2-benzothiazin-3-carboxamid-1,1-dioxid der Formel

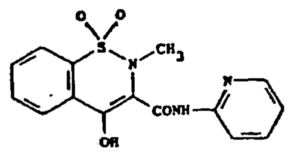

ist eine bekannte, antiinflammatorisch wirksame Substanz, die wegen ihrer schlechten Löslichkeit bei der Konfektionierung zu Lösungen Schwierigkeiten bereitet.

Die DE-A-32 17 315 stellt fest, daß aus diesem Grunde Oxicam-Derivate, zu denen das Piroxicam gehört, bis dahin nur auf oralem Weg appliziert werden konnten, und daß auch durch Salzbildung mit äquimolaren Mengen N-Methyl-D-glucamin, die für bestimmte Oxicam-Derivate aus der EP-B-2482 bekannt ist, nur eine schwache Verbesserung der Löslichkeit von Piroxicam erreicht werden könne. Darüber hinaus bestünde bei Oxicam-Derivaten das gravierende Problem der Instabilität von deren Lösungen die dazu führt, daß, selbst wenn man solche Verbindungen oder deren Salze einmal in Lösung gebracht habe,solche Lösungen dazu neigen, bereits nach relativ kurzer Zeit die Wirkstoffe wieder auszuscheiden. Die DE-A-32 17 315 schlägt nun vor, den von der Herstellung von organischen Salzen bekannten stöchiometrischen Basenanteil von 1:1 zu erhöhen und gegebenenfalls gleichzeitig ein physiologisch unbedenkliches organisches, mit Wasser mischbares Lösungsmittel zuzugeben, wodurch hochkonzentrierte Lösungen von Piroxicam mit einem Gehalt von bis zu 30% erhalten werden könnten, die selbst nach langer Lagerung stabil bleiben und keine Wirkstoffabscheidung zeigen; als Base wird insbesondere Methylglucamin vorgeschlagen.

Die DE-A-32 17 315 offenbart daher Piroxicam-haltige Arzneimittelzubereitungen, die dadurch gekennzeichnet sind, daß sie, bezogen auf den Wirkstoff, eine mehr als einmolare Menge einer organischen Base, insbesondere Methylglucamin, enthalten.

Der vorliegenden Erfindung liegt die Erkenntnis zugrunde, daß Lösungen von Piroxicam, insbesondere verdünnte Lösungen von Piroxicam, eine chemische Instabilität zeigen, d.h. daß Piroxicam leicht chemisch durch Hydrolyse und/oder Oxidation angegriffen wird, wobei als Zersetzungsprodukt 2-Aminopyridin ermittelt wurde, außerdem wurde ein Geruch nach Pyridin festgestellt.

Obwohl die Anmelderin gefunden hatte, daß bei geeigneten Lösungsmittelgemischen und einer richtigen Einstellung des pH-Wertes die Löslichkeit von Piroxicam ausreichend ist um Injektionslösungen herzustellen, bereitete die mangelhafte chemische Stabilität von Piroxicam in solchen Lösungen Prob leme.

Es wurde nun überraschenderweise gefunden, daß der Zusatz einer geringen, unterstöchiometrischen Menge von D(-)-N-Methylglucamin die chemische Stabilität von Lösungen von Piroxicam wesentlich verbessert.

Gegenstand der vorliegenden Erfindung ist daher zunächst die Verwendung von D(-)-N-Methylglucamin in unterstöchiometrischer Menge zur Verbesserung der chemischen Stabilität von Lösungen von Piroxicam.

Das Methylglucamin wird insbesondere in Mengen von 0,2 bis 0,9, vorzugsweise 0,5 bis 0,9 Mol je Mol Piroxicam eingesetzt.

Die Anmelderin hat weiter gefunden, daß sich Piroxicam in einem organisch-wässrigen Lösungsmittel gut lösen läßt, wenn der pH-Wert des Lösungsmittels auf 8 bis 9 abgepuffert ist.

Als organisch-wässriges Lösungsmittel eignen sich insbesondere Mischungen von organischen Lösungsmitteln und Wasser, die 40 bis 60 Vol.-% Wasser enthalten.

Als organische Lösungsmittel kommen die üblichen, insbesondere für Injektionslösungen geeigneten Lösungsmittel in Frage, wie z.B. Propylenglycol, Polyäthylenglycol, Dimethylformamid, Dimethylacetamid, Äthanol und andere mit Wasser mischbare, physiologisch unbedenkliche organische Lösungsmittel.

Als besonders geeignet hat sich eine Mischung von 1,2-Propandiol und Äthanol herausgestellt, wobei das Lösungsmittel 30 bis 50 Vol.-% Propandiol und 5 bis 15 Vol.-% Äthanol enthalten kann.

Das am meisten bevorzugte Lösungsmittel für eine Injektionslösung besteht aus etwa 50 Vol.-% Wasser, etwa 40 Vol.-% Propandiol und etwa 10 Vol.-% Äthanol.

In einem solchen Lösungsmittelgemisch ist Piroxicam bei einem pH-Wert von 8 weit über der benötigten Konzentration löslich. Voraussetzung ist allerdings, daß die Lösung gut gepuffert ist und in sehr engen Grenzen auf einen pH-Wert von 8 bis 9 eingestellt ist, da geringfügige pH-Verschiebungen in Richtung auf pH 7 eine starke Verringerung der Löslichkeit und eventuelle Auskristallisation mit sich bringen.

Es ist daher zweckmäßig, das Piroxicam zunächst mit Natronlauge in Lösung zu bringen und mit einem Phosphatpuffer zu versetzen, um einen pH-Wert der Piroxicamlösung von 8 bis 9 einzustellen.

Im Challenge-Test (Wechsel zwischen 5 °C und 25 °C) sind solche Lösungen über lange Zeit physikalisch stabil. Es konnten keine Auskristallisationen beobachtet werden, selbst nach 12-monatiger Lagerung im Kühlschrank (6 °C) konnte keine Auskristallisation festgestellt werden.

Dagegen bereitete die chemische Stabilität dieser Lösungen Probleme. Diese wurden nun erfindungsgemäß durch den Zusatz von D(-)-N-Methylglucamin in unterstöchiometrischer Menge überwunden.

Aus den nachstehend beschriebenen Vergleichsversuchen ist der überraschende stabilisierende Effekt des Methylglucamins auf solche Piroxicam-Lösungen ersichtlich:

Es wurden Ampullen mit einer Injektionslösung von Piroxicam gefüllt. Das Lösungsmittel bestand aus 40% Propandiol, 10% Äthanol und 50% Wasser, in welchem nach der vorstehend beschriebenen Prozedur 20 mg Piroxicam je ml Lösungsmittel gelöst wurden. Diese Injektionslösung wurde einmal ohne Zusatz von Methylglucamin, das andere Mal mit einem Zusatz von 0,9 Mol Methylglucamin je Mol Piroxicam untersucht.

Die Ampullen wurden bei 75 °C und bei 50 °C gelagert und der Gehalt der Injektionslösung an 2-Aminopyridin und der Geruch der Injektionslösung wurde nach bestimmten Lagerzeiten überprüft. Folgende Ergebnisse wurden erhalten:

Gehalt an 2-Aminopyridin in Ampullen ohne N-Methylglukamin

| Temp. | Lagerzeit in Wochen | | | |
|---|---|---|---|---|
| | 3 | 6 | 12 | |
| 50 °C | 0,57 % | 0,67 % | 0,9 % | |
| 75 °C | 2,31 % | 7,57 % | 10,5 % | Nach 12 Wochen wurden noch 63 % Piroxicam gefunden. |

Es wurde beim Öffnen der Ampullen Pyridin-Geruch festgestellt.

Gehalt an 2-Aminopyridin in Ampullen mit Zusatz von N-Methylglukamin

| Temp. | Lagerzeit in Wochen | | | |
|---|---|---|---|---|
| | 3 | 6 | 12 | |
| 50 °C | 0,01 % | 0,0 % | 0,0 % | |
| 75 °C | 0,66 % | 2,66 % | 4,0 % | Nach 12 Wochen wurden noch 86 % Piroxicam gefunden. |

Beim Öffnen der Ampullen konnte kein Pyridingeruch festgestellt werden.

Wie aus den Daten hervorgeht, sind die Ampullen, die mit N-Methylglucamin gefertigt wurden, chemisch deutlich stabiler als ohne den Zusatz.

Wie weitere Versuche zeigten, sind die stabilisierten Ampullen sogar 20 Minuten bei 120 °C sterilisierbar.

Die Vergleichsversuche zeigen die vorteilhafte Wirkung eines Zusatzes einer geringen Menge von Methylglucamin zur Verbesserung der chemischen Stabilität von Piroxicamlösungen.

Die erfindungsgemäßen Injektionslösungen enthalten im allgemeinen 1 bis 8, vorzugsweise 2 bis 4 Gew.-% Piroxicam und 0,2 bis 0,9, vorzugsweise 0,5 bis 0,9 Mol Methylglucamin je Mol Piroxicam.

Die nachstehenden Beispiele erläutern die Erfindung.

Beispiel 1:

| | | |
|---|---|---|
| Piroxicam | (1) | 20,0 g |
| Natriumdihydrogenphosphat-1-hydrat | (2) | 2,5 g |
| D(-)-N-Methylglucamin | (3) | 10,0 g |
| Propylenglykol | (4) | 400,0 g |
| Äthanol | (5) | 100,0 g |
| Natriumhydroxid | (6) | 1,0 g |
| bidest. Wasser ad | (7) | 1 l |

400 ml bidest. Wasser werden vorgelegt und unter Rühren hierin Natriumhydrogenphosphat und Methylglucamin gelöst. Zu dieser Lösung wird unter weiterem Rühren Propylenglykol und Äthanol gegeben. In der resultierenden Lösung wird Piroxicam unter Rühren gelöst. Anschließend wird der pH-Wert mit Natronlauge auf pH 8 eingestellt. Im Meßkolben wird mit bidest. Wasser auf ein Volumen von 1 Liter aufgefüllt.

Die Lösung wird mit Stickstoff durch geeignete Filter sterilfiltriert und anschließend in Ampullen abgefüllt.

Während des Lösungsvorganges und der Abfüllung der Ampullen kann mit Stickstoff zusätzlich begast werden.

Beispiel 2:

| | | |
|---|---|---|
| Piroxicam | (1) | 20,0 g |
| Natriumdihydrogenphosphat-1-hydrat | (2) | 2,5 g |
| D(-)-N-Methylglucamin | (3) | 2,5 g |
| Propylenglykol | (4) | 400,0 g |
| Äthanol | (5) | 100,0 g |
| Natriumhydroxid | (6) | 2,4 g |
| bidest. Wasser ad | (7) | 1 l |

400-ml bidest. Wasser werden vorgelegt und hierin unter Rühren das Natriumhydrogenphosphat und das N-Methylglucamin gelöst. Zu dieser Lösung wird unter weiterem Rühren Propylenglykol und Äthanol gegeben. In der resultierenden Lösung wird das Piroxicam unter Rühren gelöst.

Anschließend wird der pH-Wert mit Natronlauge auf pH 8 eingestellt. Im Meßkolben wird mit bidest. Wasser auf ein Volumen von 1 Liter aufgefüllt. Die Lösung wird mit Hilfe von Stickstoff durch geeignete Filter sterilfil

triert und anschließend in Ampullen abgefüllt. Während des Lösungsvorganges und der Abfüllung der Ampullen kann zusätzlich mit Stickstoff begast werden.

## Ansprüche

1. "Verwendung von D(-)-N-Methylglucamin in einer Menge von 0,2 - 0,9, vorzugsweise 0,5 - 0,9 Mol D(-)-N-Methylglucamin je Mol Piroxicam als chemischer Stabilisator von Lösungen von Piroxicam"

2. Injektionslösung bestehend aus einer 1-8, vorzugsweise 2-4 Gew.-%igen Lösung von Piroxicam in einem organisch-wässrigen Lösungsmittel, welches je Mol Piroxicam 0,2-0,9, vorzugsweise 0,5-0,9 Mol D(-)-N-Methylglucamin enthält.

3. Injektionslösung nach Anspruchs 2, dadurch gekennzeichnet, daß das Lösungsmittel auf einen pH-Wert von 8-9 abgepuffert ist.

4. Injektionslösung nach einem der Ansprüche 2 und 3, dadurch gekennzeichnet, daß das Lösungsmittel 40-60 Vol.-% Wasser enthält.

5. Injektionslösung nach einem der Ansprüche 2 bis 4, dadurch gekennzeichnet, daß das Lösungsmittel 30-50 Vol.-% Propandiol und 5-15 Vol.-% Äthanol enthält.

6. Injektionslösung nach einem der Ansprüche 2 bis 5, dadurch gekennzeichnet, daß das Lösungsmittel aus 50 Vol.-% Wasser, 40 Vol.-% Propandiol und 10 Vol.-% Äthanol besteht.

Patentantsprüche für folgenden Vertragsstaat: AT

1. "Verwendung von D(-)-N-Methylglucamin in einer Menge von 0,2 - 0,9, vorzugsweise 0,5 - 0,9, Mol D(-)-N-Methylglucamin je Mol Piroxicam als chemischer Stabilisator von Lösungen von Piroxicam".

2. Verfahren zur Herstellung einer Injektionslösung, dadurch gekennzeichnet, daß man 1-8 vorzugsweise 2-4 Gewichtsteile Piroxicam mit je Mol Piroxicam 0,2-0,9, vorzugsweise 0,5-0,9 Mol D(-)-N-Methylglucamin und einem organisch-wässrigen Lösungsmittel zur Herstellung von 100 Gewichtsteilen einer Injektionslösung vermischt.

3. Verfahren nach Anspruch 2, dadurch gekennzeichnet, daß das Lösungsmittel auf einen pH-Wert von 8-9 abgepuffert wird.

4. Verfahren nach einem der Ansprüche 2 und 3, dadurch gekennzeichnet, daß das Lösungsmittel 40-60 Vol.-% Wasser enthält.

5. Verfahren nach einem der Ansprüche 2 bis 4, dadurch gekennzeichnet, daß das Lösungsmittel 30-50 Vol.-% Propandiol und 5-15 Vol.-% Äthanol enthält.

6. Verfahren nach einem der Ansprüche 2 bis 5, dadurch gekennzeichnet, daß das lösungsmittel aus 50 Vol.-% Wasser, 40 Vol.-% Propandiol und 10 Vol.-% Äthanol besteht.

## Claims

1. Use of D(-)-N-methylglucamine in an amount of 0,2 - 0,9, preferably 0,5 - 0,9 mol D(-)-N-methylglucamine per mol piroxicam as chemical stabilizer of solutions of piroxicam.

2. Injection solution consisting of a 1 - 8, preferably 2 - 4 % by weight solution of piroxicam in an organic-aqueous solvent which comprises 0,2 - 0,9, preferably 0,5 - 0,9 mol D(-)-N-methylglucamine per mol piroxicam.

3. Injection solution according to claim 2, characterized in that the solvent is buffered to a pH value of 8 - 9.

4. Injection solution according to one of claims 2 and 3, characterized in that the solvent contains 40 - 60 % by volume of water.

5. Injection solution according to one of claims 2 to 4, characterized in that the solvent contains 30 - 50 % by volume of propanediol and 5 - 15 % by volume of ethanol.

6. Injection solution according to one of claims 2 to 5, characterized in that the solvent consists of 50 % by volume of water, 40 % by volume of propanediol and 10 % by volume of ethanol.

Claims for the following Contracting State: AT

1. Use of D(-)-N-methylglucamine in an amount of 0,2 - 0,9, preferably 0,5 - 0,9 mol D(-)-N-methylglucamine per mol piroxicam as chemical stabilizer of solutions of piroxicam.

2. Process for the production of an injection solution, characterized in that 1 - 8, preferably 2 - 4 parts by weight of piroxicam are mixed with per mol piroxicam 0,2 - 0,9, preferably 0,5 - 0,9 mol D(-)-N-methylglucamine and an organic-aqueous solvent for the production of 100 parts by weight of an injection solution.

3. Process according to claim 2, characterized in that the solvent is buffered to a pH value of 8 - 9.

4. Process according to one of claims 2 and 3, characterized in that the solvent contains 40 - 60 % by volume of water.

5. Process according to one of claims 2 to 4, characterized in that the solvent contains 30 - 50 % by volume of propanediol and 5 - 15 % by volume of ethanol.

6. Process according to one of claims 2 to 5, characterized in that the solvent consists of 50 % by volume of water, 40 % by volume of propanediol and 10 % by volume of ethanol.

**Revendications**

1. Utilisation de la D(-)-N-méthylglucamine en une guantité de 0,2 à 0,9, de préférence de 0,5 à 0,9 mole de D(-)-N-méthylglucamine par mole de piroxicame comme agent chimique pour la stabilisation de solutions du piroxicame.

2. Solution injectable, constituée d'une solution à 1-8, de préférence 2-4 % en poids de piroxicame dans un solvant organique-aqueux qui contient par mole de piroxicame 0,2 à 0,9, de préférence 0,5 à 0,9 mole de D(-)-N-méthylglucamine.

3. Solution injectable suivant la revendication 2, caractérisée en ce que le solvant est tamponné à une valeur de pH de 8 à 9.

4. Solution injectable suivant l'une des revendications 2 et 3, caractérisée en ce que le solvant contient 40 à 60 % en volume d'eau.

5. Solution injectable suivant l'une des revendications 2 à 4, caractérisée en ce que le solvant contient 30 à 50 % en volume de propanediol et 5 à 15 % en volume d'éthanol.

6. Solution injectable suivant l'une des revendications 2 à 5, caractérisée en ce que le solvant est constitué de 50 % en volume d'eau, 40 % en volume de propanediol et 10 % en volume d'éthanol.

Revendications pour l'Etat contractant suivant : AT

1. Utilisation de la D(-)-N-méthylglucamine en une quantité de 0,2 à 0,9, de préférence de 0,5 à 0,9 mole de D(-)-N-méthylglucamine par mole de piroxicame comme agent chimique pour la stabilisation de solutions du piroxicame.

2. Procédé de préparation d'une solution injectable, caractérisé en ce qu'on mélange 1 à 8, de préférence 2 à 4 parties en poids de piroxicame avec, par mole de piroxicame, 0,2 à 0,9, de préférence 0,5 à 0,9 mole de D(-)-N-méthylglucamine et d'un solvant organique-aqueux pour la préparation de 100 parties en poids d'une solution injectable.

3. Procédé suivant la revendication 2, caractérisé en ce que le solvant est tamponné à une valeur de pH de 8 à 9.

4. Procédé suivant l'une des revendications 2 et 3, caractérisé en ce que le solvant contient 40 à 60 % en volume d'eau.

5. Procédé suivant l'une des revendications 2 à 4, caractérisé en ce que le solvant contient 30 à 50 % en volume de propanediol et 5 à 15 % en volume d'éthanol.

6. Procédé suivant l'une des revendications 2 à 5, caractérisé en ce que le solvant est constitué de 50 % en volume d'eau, 40 % en volume de propanediol et 10 % en volume d'éthanol.